# EUROPÄISCHE PATENTSCHRIFT

(11) **EP 1 466 614 B1**
(45) Veröffentlichungstag und Bekanntmachung des Hinweises auf die Patenterteilung: **15.10.2008**
(21) Anmeldenummer: 02796999.7
(22) Anmeldetag: 20.12.2002
(51) Int. Cl.: A61K 47/48, C07K 7/06, A61K 31/704, A61P 35/00, A61K 38/08

(54) **POLYPEPTID, DESSEN KONJUGAT DOXORUBICIN ENTHÄLT, UND AUF DIESEM BASIERENDE PHARMAZEUTISCHE ZUSAMMENSETZUNG**
POLYPEPTIDE, THE CONJUGATE THEREOF CONTAINING DOXORUBICINE AND A PHARMACEUTICAL COMPOSITION BASED THEREON
POLYPEPTIDE, SON CONJUGUE AVEC DOXORUBICINE ET COMPOSITION PHARMACEUTIQUE SUR CETTE BASE

(30) Priorität: 21.12.2001 RU 2001134536
(43) Veröffentlichungstag der Anmeldung: 13.10.2004
(73) Patentinhaber: ZAKRYTOE AKTSIONERNOE OBSCHESTVO "RUSSKIE BIOTECHNOGII", Moscow, 113054 (RU)
(72) Erfinder: SEVERIN, Evgeniy Sergeevich, Moscow, 125047 (RU); SEVERIN, Sergey Evgenyevich, Moscow, 117418 (RU); LUTSENKO, Sergey Viktorovich, Moscow, 119021 (RU); KISELEV, Sergey Michailovich, 111531 Moscow (RU); FELDMAN, Natalya Borisovna, 300041 Tula (RU)
(74) Vertreter: Jeck, Anton
(86) Internationale Anmeldenummer: PCT/RU2002/000544
(87) Internationale Veröffentlichungsnummer: WO 2003/053457

(56) Entgegenhaltungen:
- EP-A2- 0 441 218
- WO-A-85/01284
- RU-C1- 2 107 690
- US-A- 5 824 805
- US-A- 5 969 099
- DE ACOSTA M ET AL: "Preparation and in vitro toxicity between a conjugate of epidermal growth factor and adriamycine" INTERFERON Y BIOTECNOLOGIA 1989 CUBA, Bd. 6, Nr. 2, 1989, Seiten 177-185, XP008062055 ISSN: 0138-8878
- KISELEV, S. M. [REPRINT AUTHOR] ET AL: "Cytotoxic activity of the conjugate of the epidermal growth factor with doxorubicin against human cells." TUMOR BIOLOGY, (SEPTEMBER, 2000) VOL. 21, NO. SUPPLEMENT 1, PP. 137. PRINT. MEETING INFO.: 28TH MEETING OF THE INTERNATIONAL SOCIETY FOR ONCODEVELOPMENTAL BIOLOGY AND MEDICINE. MUNICH, GERMANY. SEPTEMBER 08-13, 2000. INTERNATIONAL SOCIETY FOR ONCODEV, September 2000 (2000-09), XP008062070
- LUTSENKO S.V. ET AL: 'Stimulation of cytotoxic activity of doxorubicin in its transport to tumor cells with protein vehicles' RUSSIAN JOURNAL OF ONCOLOGY Bd. 3, 2001, Seiten 33 - 37, XP008071879

## Beschreibung

### Erfindungsgebiet

Die Erfindung bezieht sich auf das Gebiet der Chemie der Naturstoffe, der Pharmakologie und der Medizin und kann in der Medizin für die Behandlung von Krebskranken verwendet werden.

### Stand der Technik

Unter den Hauptgründen, die wesentlich die Wirksamkeit der antiblastomatösen Chemotherapie begrenzen, kann die protopathische und erworbene Resistenz der Krebszellen gegen Medikamente und die niedrige Selektivität ihrer Wirkung gezählt werden. Die Erhöhung der Selektivität der Chemomittel kann durch deren zielgerichteten Transport zu den Zielzellen mittels unterschiedlicher Eiweißvektoren erreicht werden. Dabei gelangen die Chemomittel in die Zelle aufgrund der rezeptorvermittelten Endozytose der Zusammensetzung, die die kovalente Verbindung von Eiweiß und Chemomitteln darstellt. Die Selektivität der Wirkung von Zusammensetzungen wird entweder durch das Vorhandensein von spezifischen Rezeptoren auf der Oberfläche der Geschwulstzellen, wobei die Rezeptoren vom Vektoreiweiß oder vom Immunkörper "erkannt" werden, oder durch ein höheres Niveau der Vektorproteinrezeptoren auf der Oberfläche der Krebszellen im Vergleich zu den normalen Zellen erreicht. Die Möglichkeit der wirksamen Verwendung des epidermalen Wachstumsfaktors (EGF) als Vektormolekül ist durch dessen hohe Stabilität, die Zugänglichkeit als rekombinantes Eiweiß und durch einen höheren Gehalt der EGF-Rezeptoren auf der Oberfläche der Geschwulstzellen im Vergleich zu den normalen Zellen bedingt.

Eine große Anzahl von Studien und Patenten ist bekannt, die den verschiedenen Zusammensetzungen auf der Basis des epidermalen Wachstumsfaktors (EGF) mit Adriamycin und anderen antiblastomatösen Mitteln, den Verfahren zu ihrer Gewinnung und einer pharmazeutischen Komposition auf ihrer Basis gewidmet sind (US-Patente 5824805, 5349066, 5087616, 5393737, 5505931 5622929, 5122368, 5776458 usw.).

Adriamycin, ein bekanntes antiblastomatöses Antibiotikum, hat trotz einer breiten klinischen Verwendung eine Reihe von wesentlichen Nachteilen, zu denen eine hohe Herzgiftigkeit und eine sich schnell entwickelnde Resistenz der Krebszellen bei wiederholten, intravenösen Injektionen des Antibiotikums gehört. Die Verwendung von Adriamycin könnte in der Zusammensetzung dessen therapeutischen Eigenschaften bedeutend verbessern.

Ein besonderes Interesse findet die Verwendung von kurzen EGF-Peptidfragmenten als Vektormoleküle für den Transport von Adriamycin und anderen antiblastomatösen Mitteln in die Geschwulstzielzellen. Da die Technologie ihrer Gewinnung relativ einfach und wirtschaftlich ist und die Verwendung des biologischen Rohstoffs für ihre Herstellung nicht voraussetzt, lassen sich die auf ihrer Basis geschaffenen Zusammensetzungen leichter standardisieren.

Bekannt ist für die Krebs-Chemotherapie die Verwendung von Mischungen (pharmazeutischen Kompositionen) des Adriamycins (und anderen antiblastomatösen Mitteln) und verschiedenen EGF-Peptidfragmenten (US-Patent 4863902). Die Steigerung der antiblastomatösen Wirkung der Mittel gemäß der vorliegenden Erfindung erklären die Autoren durch eine höhere Empfindlichkeit der Geschwulstzellen auf die Adriamycin-Wirkung vor dem Hintergrund der Stimulierung der Zellenproliferation, die durch den epidermalen Wachstumsfaktor und seine aktiven Fragmente vermittelt wird. Die nicht kovalente Immobilisierung von Adriamycin mit den EGF-Peptidfragmenten und EGF garantieren den zielgerichteten Transport zu den Geschwulstzielzellen nicht und erhöhen damit die nichtspezifische Giftigkeit des Mittels.

In der Krebstherapie ist auch die Verwendung von biologisch aktiven, zyklischen Polypeptiden bekannt, die eine Aminosäuresequenz aufweisen, die der Sequenz der 32. bis 48. EGF-Aminosäure analog ist, s. US-Patent 5183805. In diesem Patent ist angegeben, dass das Peptid chemisch mit chemotherapeutischen Wirkstoffen, wie z.B. Adriamycin, zusammengesetzt werden kann, aber die Zusammensetzungen sind im Patentanspruch nicht angegeben, die Beschreibung der Herstellung dieser Zusammensetzungen fehlt auch, und die biologischen Eigenschaften sind nicht angegeben.

Ferner ist ein Polypeptid bekannt, das ein Fragment des epidermalen Wachstumsfaktors der 21. bis 31. Aminosäure der Formel

W₂N-MYIEALDKYAC-COOH (1)

ist, das sich wirksam mit dem EGF-Rezeptor verbinden und die Proliferation der Zellen hervorrufen kann; seine Zusammensetzung mit Adriamycin erfolgt im Verhältnis von 1:1, wobei die zusammengesetzten Teile mit säurelabilem Azomethan mit Hilfe von Glutardialdehyd als Verbindungsmittel verbunden sind (s. das russische Onkologiemagazin, Nr. 3, 2001, Seiten 33-37, "Die Erhöhung der zytotoxischen Aktivität von Adriamycin bei seinem Transport in die Geschwulstzellen durch Eiweißvektoren"). Als pharmazeutische Kompositionen können beliebige Kompositionen angegeben werden, die in den US-Patenten 5349066, 5087616, 5122368 beschrieben sind und die Adriamycin-Zusammensetzungen mit EGF in einer wirksamen Menge sowie einen für die intravenöse Injektion geeigneten, pharmazeutischen Träger enthalten.

### Kurzfassung der Erfindung

Die Aufgabe der vorliegenden Erfindung besteht darin, die Palette der Polypeptide, deren Zusammensetzung mit Adriamycin und deren pharmazeutische Komposition zu erweitern und deren Wirksamkeit zu erhöhen.

Diese Aufgabe wird dadurch gelöst, dass ein neues Polypeptid vorgeschlagen wird, das eine Analogie des Fragments des epidermalen Wachstumsfaktors von der 21. bis 31. Aminosäure ist, das fähig ist, sich wirksam mit dem Rezeptor des epidermalen Wachstumsfaktors zu verbinden, die Proliferation der Zellen hervorzurufen und die Funktion des Vektors für den zielgerichteten Transport von antiblastomatösen Mitteln in die Krebszellen zu erfüllen. Das Polypeptid hat folgende Struktur:

H₂N-MYIEALDSYAC-COOH (2).

Im Gegensatz zum Polypeptid der Formel (1) ist im Polypeptid gemäß der Erfindung die Lys-Aminosäure, die sich im aktiven Zentrum des rezeptorverbindenden Fragments des menschlichen EGF's befindet, durch die Ser-Aminosäure ersetzt, die das Verbindungszentrum im Maus-EGF bildet. Es wurde unerwartet festgestellt, dass das Ersetzen von Lys gegen Ser erlaubt, eine unerwünschte Konjugierung von Lysin mit ε-Aminogruppen im aktiven Zentrum des rezeptorverbindenden Fragments zu vermeiden, wodurch wiederum die Hemmung der Verbindung des Polypeptids mit dem EGF-Rezeptor verhindert wird.

Die Wirksamkeit des neuen Polypeptids ist durch die Verwendung des bekannten Antibiotikums Adriamycin begründet, aber es können andere antiblastomatöse Antibiotika verwendet werden, wie Vinkristin, Cisplatin, Daunomycin, Methotrexat sowie weitere bekannte, antiblastomatöse Mittel: Toxine wie Diphtherietoxin oder Rizin, Zyklophosphamid usw.

Eine weitere Aufgabe der vorliegenden Erfindung besteht ferner darin, eine Polypeptid-Zusammensetzung mit Adriamycin zu schaffen, in der Adriamycin kovalent am Polypeptid durch Azomethan gebunden ist, wobei das Polypeptid die Formel MYIEALDSYAC aufweist. Diese Polypeptidzusammensetzung besitzt eine selektive Wirkung im Hinblick auf die Krebsgeschwülste und kann die Resistenz der Geschwulstzellen gegen die antiblastomatösen Mittel bedeutend vermindern.

Als Verbindungsmittel zu den Aminogruppen des Polypeptids und des Adriamycins kann ein Glutaraldehyd und andere bekannte bifunktionelle Mittel verwendet werden. Als Ergebnis wird eine Zusammensetzung mit einer säurelabilen, chemischen Verbindung erhalten.

Gegenstand dieser Erfindung ist auch eine pharmazeutische Komposition, die eine antiblastomatöse Wirkung auf der Basis der vorgeschlagenen Zusammensetzung des rezeptorverbindenden Fragments des epidermalen Wachstumsfaktors mit Adriamycin in einer wirksamen Menge (von 0,05-0,1%) und einen annehmbaren Träger für die intravenöse Injektion aufweist.

Dabei können als Träger eine physiologische Lösung, eine Phosphat-Salz-Lösung oder ein Schmerzminderungsmittel auftreten. Die Komposition kann auch zusätzlich mikrobizide, viruzide und antiparasitäre Mittel sowie andere Mittel enthalten, die für die Behandlung von Krebskranken eingesetzt werden, und zwar im Umfang von nicht mehr als 50% des Gesamtgewichts der Komposition.

### Kurzbeschreibung der Zeichnungen

Die Erfindung wird nun anhand von Ausführungsbeispielen näher beschrieben. Es zeigen:
- Fig. 1: Vergleichsdaten in Bezug auf den Einfluss des vorgeschlagenen und bekannten Polypeptids der Formel (1) auf die Proliferation der Fibroblastzellen der Maus der Linie NIH 3T3 und
- Fig.2: Angaben über die Verzögerung der Erscheinungszeit von Geschwülsten und über die Geschwindigkeit des Geschwulstwachstums unter dem Einfluss von adriamycinhaltigen Zusammensetzungen am Modell der übertragbaren Mausgeschwülste.

### Beschreibung der Ausführungsbeispiele

Unten sind Beispiele angeführt, die die vorliegende Erfindung und die Möglichkeit der Verwendung des rezeptorverbindenden Fragments des epidermalen Wachstumsfaktors (EGF), der vorgeschlagenen Zusammensetzung und der Komposition auf seiner Basis zu dem genannten Verwendungszweck illustrieren.

### Beispiel 1: Gewinnung des Polypeptids

Das Polypeptid wurde durch ein Verfahren der phasenharten Peptidchemie unter Verwendung der Fmoc-Synthesestrategie gewonnen. Als Kunststoffträger wurde das Harz Wang-resin ausgewählt. Bei jedem Schritt der Peptidkondensation wurde die Karboxylgruppe von Fmoc-Aminosäuren durch die Umwandlung in Hydroxidbenzolazimidester mit Hilfe von HOBt und N.N-Diisopropylkarbodiimid der Fmoc-Aminosäure aktiviert. HOBt und N.N-Diisopropylkarbodiimid wurden in 10-fachen molaren Überschüssen in Bezug auf die Aminogruppen des Polymers in Reaktion gebracht. Der Ablauf der Reaktion wurde durch die Veränderung der Farbe von blauem Bromophenol verfolgt, das in den Reaktor als Lösung in Dimethylsulfoxid (DMCO) im Verhältnis von 1/1000 (mol) zur Menge der Aminogruppe zugefügt wurde. Die Reaktion dauerte 1,5-2 Stunden. Nach der Beendigung der Kondensation wurde Harz viermal mit Dimethylformamid (DMF) gespült und mit einer 50%igen Piperidinlösung in Dimethylformamid bearbeitet. Die Polypeptidabspaltung vom Kunststoffträger wurde mit einer Mischung aus Trifluoressigsäure zu 95%, Wasser zu 2,5% und 1,2-Äthandithiol zu 2,5% durchgeführt. Nachdem das Harz gefiltert worden war, wurde das Peptid aus der Lösung durch Diethylether gefällt. Der Niederschlag wurde durch Zentrifugieren abgetrennt, im Vakuum getrocknet und durch das HPLC-Verfahren mit Phasenumkehr auf der Säule Phenomenex Luna C18 250*3 analysiert.

Die Gradientelution des Polypeptids aus der Säule wurde in einem System von Lösungsmitteln durchgeführt: Azetonitril zu 0,1 % von Trifluoressigsäure/Wasser.

Das halbpräparative HPLC-Verfahren mit Phasenumkehr wurde unter gleichen Bedingungen auf der Säule Nucleosil C18 250*8 durchgeführt. Die Frequenz des Polypeptids nach der chromatographischen Trennung betrug >96%.

Das Polypeptid wurde durch das Verfahren der Massenspektrometrie gekennzeichnet (Molekülion = 1265). Die Struktur des Polypeptids ist in Tabelle 1 angeführt.

**Tabelle 1**

| |
|---|
| Die Struktur des bekannten und des vorgeschlagenen Polypeptids |
| Die Aminosäuresequenz des Polypeptid-Prototyps |
| NH₂-Met-Tyr-Ile-Glu-Ala-Leu-Asp-Lys-Tyr-Ala-Cys-COOH (ε-NH₂) |
| Die Aminosäuresequenz des vorgeschlagenen Polypeptids |
| NH₂-Met-Tyr-Ile-Glu-Ala-Leu-Asp-Ser-Tyr-Ala-Cys-COOH |

### Beispiel 2: Gewinnung der Polypeptid-Zusammensetzung mit Adriamycin

Das Polypeptid wurde in 5ml 0,05M-Phosphatpuffer (pH7; Konzentration 1mg/ml) aufgelöst; dann wurde Adriamycin (Doxorubicin DR) hinzugefügt, das vorher in 5ml destilliertem Wasser (pH=5,5; Konzentration 1,2mg/ml) aufgelöst wurde. Die Synthese der Zusammensetzung wurde nach dem Schema 1 realisiert.

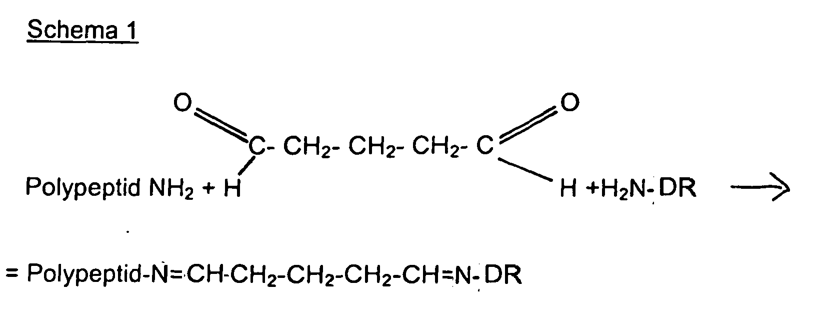

Zu der Reaktionsmischung wurde beim Umrühren tropfenweise Glutardialdehyd (GA) in einem 0,05M-Phosphatpuffer (pH=7,2; Konzentration GA-0.5 mg/ml) zugefügt. Der Umfang der zugefügten GA-Mischung betrug 3ml. Nach dem Zufügen der GA-Mischung wurde die Mischung im Laufe von einer Stunde bei 50°C inkubiert. Die synthetisierte Zusammensetzung wurde von den Ausgangsstoffen auf der Säule PD-10 Sephadex G-25 (Pharmacia, Schweden) abgetrennt. Die Zusammensetzung wurde im HPLC-Verfahren mit Phasenumkehr auf der Säule Nucleosil C18 250*4.6 analysiert. Die Gradientelution der Zusammensetzung aus der Säule wurde in einem System von Lösungsmitteln durchgeführt: Azetonitril zu 0,1 % von Trifluoressigsäure/Wasser.

Die Konzentration des Polypeptids in der Zusammensetzung wurde spektralphotometrisch (Wellenlänge λ=280nm) festgestellt. Der Gehalt an Adriamycin wurde spektralphotometrisch bei der Wellenlänge λ=495nm bestimmt. Das Molverhältnis Polypeptid : Adriamycin in der Zusammensetzung betrug 1:1.

Hiermit wurde ein Verfahren der chemischen Zusammensetzung des vorgeschlagenen Polypeptides und Adriamycins mit Glutardialdehyd als Verbindungsmittel entwickelt. Es wurde die Zusammensetzung Polypeptid-Adriamycin gewonnen und gekennzeichnet, in der das Verhältnis der zusammengesetzten Teile 1:1 betrug.

### Beispiel 3: Die biologische Aktivtät des Polypeptids

Die Bestimmung der proliferationsmäßigen Aktivität des Polypeptids wurde auf den Fibroblastzellen der Maus der Linie NIH 3T3 durchgeführt. Die Intensivität der DNA-Biosynthese wurde durch die Einlagerung von ³H-Thymidin in die säurelösliche Zellenfraktion festgestellt.

Die Fibroblastzellen der Maus der Linie NIH 3T3 wurden in Kunststoffflakons (Costar) im Medium RPMI (Sigma) kultiviert, das 10% embryonales Rinderserum (Sigma), 100 U/ml Penizillin und 100 µg/ml Streptomyzin in der Nassluft mit 5% CO₂ bei 37°C enthält. Die Proliferation der synchronisierten Fibroblasten der Linie NIH 3T3 wurde unter Zusatz von Polypeptid in verschiedenen Konzentrationen (0.1-1000ng/ml) zu der Suspension der Zellen induziert. Die Fibroblasten wurden auf 96-well-Mikrotiterplatten in einem 200mkl-Medium von je 200-800 Tausend/well im vollen Medium kultiviert. ³H-Tymidin (1mkKi/well, 40 mKi/Mol) wurde zwei Stunden vor der Beendigung der Inkubation zugefügt. Die Radioaktivität der Zellen wurde mit einem Flüssigkeitsszintillationszähler (Rackbeta, LKB) unter Verwendung der Szintillationsflüssigkeit 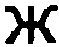 C (JS)-8 auf Toluol-Basis gemessen. Die Intensivität der DNA-Biosynthese wurde als Index der Stimulation (Verhältnis der stimulierten Zellen zu den Kontrollzellen) nach der Formel ausgedruckt: I=N/Nox100%, wobei 1 der Index der Stimulation, N die Anzahl der stimulierten EGF-Zellen und No die Anzahl der Kontrollzellen ist.

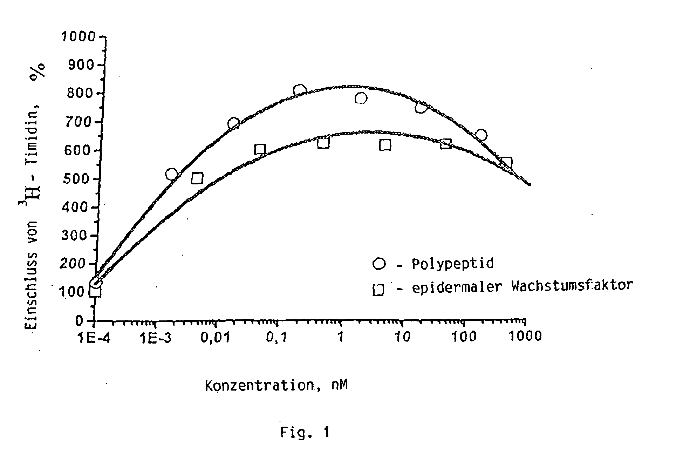

In Fig. 1 sind die Vergleichsdaten bezüglich des Einflusses des vorgeschlagenen Polypeptids der Formel 1 (EGF) auf die Proliferation der Fibroblastzellen der Maus der Linie NIH 3T3 dargestellt. Diese Angaben demonstrieren eine ausgeprägtere dosisabhängige, proliferationsmäßige Wirkung des Polypeptids im Vergleich dieser Zellenlinie zum bekannten Polypeptid; diese Wirkung kann in einer höheren Wirkung des Zusammenwirkens des Polypeptids mit dem Rezeptor begründet sein. Da die Ausdruckskraft der Rezeptoren im Verhältnis zu dem epidermalen Wachstumsfaktor um mehrere Größenordnungen diese bei normalen Körperzellen überschreitet, bestätigt die Tatsache die Möglichkeit der Verwendung des vorgeschlagenen Polypeptids als ein wirksameres Vektormolekül für den Transport der antiblastomatösen Mittel in die Geschwulstzellen.

Beispiel 4: Die Wirksamkeit der Polypeptid-Zusammensetzung mit Adriamycin im Hinblick auf Geschwulstzellen verschiedener Linien, ausgewertet nach der Überlebensfähigkeit.

Die Wirkung der Zusammensetzung wurde auf den folgenden Zellenlinien untersucht: Milchdrüsenkarzinom der MCF-7^{wt}-Linie und die Resistenzlinie des menschlichen Milchdrüsenkarzinoms MCF-7^{AdrR}; Eierstockkarzinom der SKOV3-Linie und die Resistenzlinie des Eierstockkarzinoms SKVLB; Mausmelanom der Linie B16.

Das Prüfprotokoll war allgemein für alle durchgeführten Untersuchungen und lautete folgendermaßen: die Zellen wurden in Kunststoffflakons (Costar) im Medium RPMI (Sigma) kultiviert, das 10% embryonales Rinderserum (Sigma), 100 U/ml Penizillin und 100 µg/ml Streptomyzin enthält, in der Nassluft 5% CO₂ bei 37°C kultiviert. Die Zellen wurden auf 96-well-Mikrotiterplatten (Costar) mit je 10 000 Zellen/well verteilt, indem die zu untersuchenden Mittel in verschiedenen Konzentrationen zugefügt und 72 Stunden inkubiert wurden. 2-4 Stunden vor der Beendigung der Inkubation wurde in jede Vertiefung je 50 mkl (1mg/ml) MTT-Lösung (3-[4,5 Dimethylthiazol-2-yl]-2,5 Diphenyltetrazol Bromid, Sigma) im Medium für die kultivierten Zellen zugefügt. Nach der Entwicklung der Färbung wurde das Medium entfernt, die Formazan-Kristalle wurden in 150mkl Dimethylsulfoxid aufgelöst, und spektrometrisch wurde die Intensität der Färbung nach der Absorption bei 540nm festgestellt. Die Überlebensfähigkeit der Zellen, die der Wirkung von Adriamycin und der Zusammensetzung ausgesetzt wurden, wurde in % ausgewertet, indem für 100% die Überlebensfähigkeit der Kontrollkultur angenommen wurde.

Die Angaben über die Überlebensfähigkeit der Zellen nach ihrer Inkubation mit der Polypeptid-Adriamycin-Zusammensetzung, freiem Adriamycin und mit der Zusammensetzung EGF-Adriamycin sind in der Tabelle 2 aufgeführt:

**Tabelle 2**

| Die Überlebensfähigkeit der Geschwulstrellen nach ihrer Inkubation mit freiem oder mit zusammengesetztem Adriamycin | | | |
|---|---|---|---|
| Zellenlinie | | | |
| | Adriamycin | Zusammensetzung EGF-Adriamycin | Zusammensetzung Polypeptid-Adriamycin |
| MCF-7^{Wt} | 58,6 nM | 2,5 nM | 1,3 nM |
| MCF-7^{AdrR} | 3,7 mkM | 0,3 mkM | 0,15 mkM |
| SKOV3 | 209 nM | 35 nM | 22 nM |
| SKVLB ^{AdrR} | 2,01 mkM | 0,2 mkM | 0,07 mkM |
| B16 | 35 nM | 1,5 nM | 1,1 nM |

Aus diesen Angaben folgt, dass die vorgeschlagene Polypeptid-Zusammen-setzung mit Adriamycin eine bedeutend höhere toxische Wirkung im Hinblick auf die menschlichen Geschwulstzellen der MCF-7^{Wt} und SKOV3-Linien, die auf Adriamycin empfindlich ansprechen, und im Hinblick auf die Zellen des Mausmelanoms B16 als freies Adriamycin und dessen Zusammensetzung mit EGF (Prototyp) aufweist. Die Zusammensetzung Polypeptid-Adriamycin hat auch eine höhere, zytotoxische Aktivität im Hinblick auf die Zellen der Linie MCF-7^{AdrR} und SKVLB aufgewiesen, die über die Resistenz zu den Anthracyclin-Antibiotika verfügen.

Hiermit ist gezeigt, dass das antiblastomatöse Mittel (Adriamycin) seiner Zusammensetzung mit dem vorgeschlagenen Polypeptid eine bedeutende zytotoxische Wirkung im Hinblick auf die Geschwulstzellen aufweist, die sowohl empfindlich als auch resistent im Hinblick auf Adriamycin sind, wodurch die Wirksamkeit des vorgeschlagenen Polypeptids als Vektormolekül für den Transport der antiblastomatösen Mittel unter Bedingungen in vitro bewiesen ist.

### Beispiel 5:

Die pharmazeutischen Kompositionen für die Injektionen gewinnt man durch das Auflösen der Zusammensetzung in einer physiologischen Lösung oder in einer Phosphat-Salz-Lösung. Der pH-Wert der Lösungen betrug etwa 7,4. Die Konzentration der Zusammensetzung in der Lösung betrug 0,05-0,1 %.

Beispiel 6: Die Antitumoraktivität der Komposition der Zusammensetzung Polypeptid-Adriamycin mit der physiologischen Lösung nach Beispiel 5 am Modell der übertragbaren Geschwülste der Mäuse.

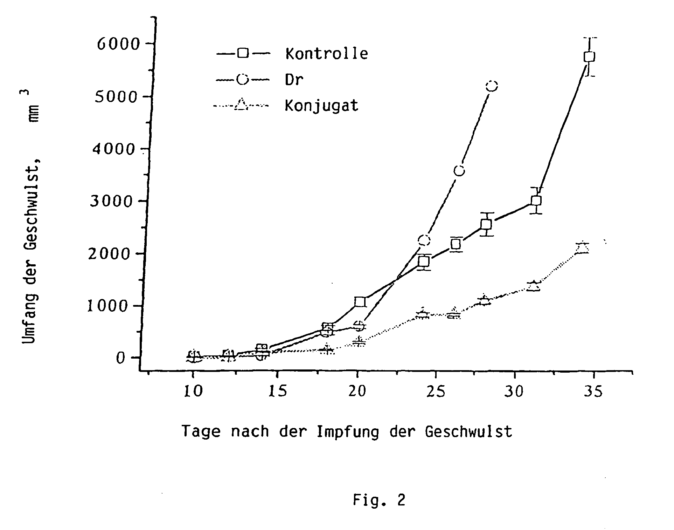

Für die Vergleichseinschätzung der therapeutischen Wirksamkeit der Zusammensetzung Polypeptid-Adriamycin im Hinblick auf feste Geschwülste, die durch die Unterhautinjektion der Krebszellen der Linie B16 bei Mäusen gewonnen werden, wurde das intravenöse Verfahren der Einführung der Kompositionen verwendet, die im Beispiel 5 beschrieben sind. Die erreichten Werte sind in der Fig. 2 und in der Tabelle 3 dargestellt. Die Präparate wurden in einer Dosis von 0,2 mg/kg Adriamycin nach dem Schema einmal pro 4 Tage eingeführt, insgesamt 3 Injektionen, beginnend vom 3. Tag nach der Impfung der Geschwulst.

Aus Figur 2 folgt, dass die Zusammensetzung im Gegensatz zum freien Adriamycin die Erscheinungszeit der Geschwülste und die Geschwindigkeit des Geschwulstwachstums bedeutend verzögert hat. Die Verzögerung des Geschwulstwachstums betrug 58%.

Aus der Tabelle 3 folgt, dass die Zusammensetzung gemäß der Erfindung eine therapeutische Wirkung bei dem intravenösen Verfahren der Injektion des Präparats erzielen kann, das nach der Erhöhung der Lebensdauer der Tiere ausgewertet wurde. Die Anwendung der Zusammensetzung führte zu einer 46%igen Erhöhung der Lebensdauer der Tiere; diese Erhöhung kann im Fall von freiem Adriamycin nicht erreicht werden.

**Tabelle 3**

| Steigerung der durchschnittlichen Lebensdauer von Tieren bei der intravenösen Injektion von adriamycinhaltigen Kompositionen | | |
|---|---|---|
| Gruppe der Tiere | Durchschnittliche Lebensdauer der Tiere, Tage | Erhöhung der durchschnittlichen Lebensdauer der Tiere % |
| Kontrolle | 31,4 + 4,8 | - |
| Adriamycin | 23,6 + 4,0 | - 24,8 |
| Zusammensetzung | 46,6 + 5,7 | 48,5 |

Die Erhöhung der durchschnittlichen Lebensdauer der Tiere sowie die höheren Kennziffern der Hemmung des Geschwulstwachstums bestätigen die therapeutische Wirksamkeit des zielgerichteten Transports des Tumormittels in der Zusammensetzung der vorgeschlagenen Komposition unter in-vivo-Bedingungen.

Die Beispiele 4 und 6 zeigen, dass die Zusammensetzung des Polypeptids gemäß der Erfindung zusammen mit dem Antibiotikum der Anthracyclin-Reihe Adriamycin (Polypeptid-Adriamycin) über eine therapeutische Wirksamkeit im Hinblick auf feste Geschwülste beim intravenösen Injektionsverfahren verfügt.

## Patentansprüche

1. Biologisch aktives Polypeptid MYIEALDSYAC, das ein Analogon des EGF-Fragments der 21. bis 31. Aminosäure darstellt und über die Fähigkeit verfügt, sich an den EGF-Rezeptor zu binden sowie eine Zellenproliferation zu bewirken, und das die Funktion eines Vektormoleküls zum zielgerichteten Transport von antiblastomatösen Präparate zu den Tumorzellen erfüllt.

2. Konjugat eines Polypeptids nach Anspruch 1 mit Doxorubicin, in dem Doxorubicin kovalent an das Peptid durch eine säurelabile Azomethinbindung gebunden ist.

3. Polypeptidkonjugat mit Doxorubicin nach Anspruch 2, in dem Doxorubicin kovalent an das Peptid unter Verwendung von Glutaraldehyd als Bindeagens gebunden ist.

4. Pharmazeutische Zusammensetzung, die über eine antiblastomatöse Wirkung verfügt und ein Konjugat nach einem der Ansprüche 2 und 3 in der wirksamen Menge sowie einen für eine intravenöse Verabreichung passenden, pharmazeutischen Träger enthält.

## Claims

1. Biologically active polypeptide MYIEALDSYAC, which represents an analogue of the EGF fragment of the 21st to 31st amino acid and has the ability to bind to the EGF receptor, and to bring about cell proliferation, and which fulfils the function of a vector molecule for targeted transport of antiblastoma products to the tumour cells.

2. Conjugate of a polypeptide according to Claim 1 with doxorubicin, in which doxorubicin is covalently linked to the peptide by an acid-labile azomethine linkage.

3. Polypeptide conjugate with doxorubicin according to Claim 2, in which doxorubicin is covalently linked to the peptide by use of glutaraldehyde as linking agent.

4. Pharmaceutical composition which has an antiblastoma effect and comprises a conjugate according to either of Claims 2 and 3 in the effective amount, and a pharmaceutical carrier suitable for intravenous administration.

## Revendications

1. Polypeptide biologiquement actif MYIEALDSYAC, qui constitue un analogue du fragment de l'EGF de l'acide aminé 21 à 31 et possède la capacité de se lier au récepteur de l'EGF et de provoquer une prolifération cellulaire, et qui joue le rôle d'une molécule vecteur pour le transport ciblé de préparations antiblastomatoses vers les cellules tumorales.

2. Conjugué d'un polypeptide selon la revendication 1 avec la doxorubicine, dans lequel la doxorubicine est reliée de manière covalente au peptide par une liaison azométhine instable en milieu acide.

3. Conjugué d'un polypeptide avec la doxorubicine selon la revendication 2, dans lequel la doxorubicine est reliée de manière covalente au peptide en utilisant le glutaraldéhyde en tant qu'agent de liaison.

4. Composition pharmaceutique, qui possède une action antiblastomatose et qui contient un conjugué selon l'une quelconque des revendications 2 et 3 en la quantité efficace, ainsi qu'un véhicule pharmaceutique approprié pour une administration intraveineuse.
